Europäisches Patentamt

European Patent Office

Office européen des brevets

(11) Publication number: **0 453 097 B1**

(19)

# EUROPEAN PATENT SPECIFICATION

(45) Date of publication of patent specification: **06.12.95**   (51) Int. Cl.⁶: **A61K 47/48**, A61K 7/28

(21) Application number: **91302404.8**

(22) Date of filing: **20.03.91**

(54) **Zusammensetzung enthaltend zumindest zwei verschiedene Antikörper oder deren Fragmente.**

(30) Priority: **21.03.90 GB 9006327**
        **05.10.90 GB 9021671**

(43) Date of publication of application:
**23.10.91 Bulletin 91/43**

(45) Publication of the grant of the patent:
**06.12.95 Bulletin 95/49**

(84) Designated Contracting States:
**AT BE CH DE DK ES FR GB GR IT LI NL SE**

(56) References cited:
EP-A- 0 315 364      EP-A- 0 361 908
WO-A-88/01178     WO-A-89/11866
WO-A-91/00112     FR-A- 2 187 294

STN FILE SERVER, FILE MEDLINE, Derwent
Publications Ltd., London (GB); R.B. JOHN-
SON, Jr. et al., AN 81192354

STN FILE SERVER, FILE CHEMICAL AB-
STRACTS, vol. 105, no. 17, abstract no.
145834w, Columbus, OH (US); J. PENE et al.,
no. 145834w

FILE WPIL, Derwent Publications Ltd., Lon-
don (GB); AN 88-043338

(73) Proprietor: **UNILEVER PLC**
**Unilever House**
**Blackfriars**
**London EC4P 4BO (GB)**

(84) Designated Contracting States:
**GB**

(73) Proprietor: **UNILEVER N.V.**
**Weena 455**
**NL-3013 AL Rotterdam (NL)**

(84) Designated Contracting States:
**BE CH DE DK ES FR GR IT LI NL SE AT**

(72) Inventor: **Beggs, Thomas Stewart**
**151 Needwood Road**
**Elms Farm,**
**Bedford MK 41 0DN (GB)**
Inventor: **Davis, Paul James**
**The Hawthorns,**
**Pavenham Road**
**Felmersham,**
**Bedford MK 43 7EX (GB)**

(74) Representative: **Ford, Michael Frederick et al**
**MEWBURN ELLIS**
**York House**
**23 Kingsway**
**London WC2B 6HP (GB)**

**Description**

FIELD AND BACKGROUND OF THE INVENTION

This invention relates to the delivery of active agents to target sites, with provision for binding to the target sites. The function of the agent will usually be therapeutic but may be cosmetic.

The use of an antibody to attach a therapeutic agent to target sites has already been proposed, with glucose oxidase as the therapeutic agent. This enzyme catalyses the oxidation of glucose to gluconic acid by molecular oxygen, producing hydrogen peroxide in the process.

Hydrogen peroxide is rapidly decomposed in vivo, but if it can be brought close to target cells it does exhibit toxicity to those cells.

SUMMARY OF THE PRIOR ART

Knowles et al, J. Clinical Investigation 52 1443 (1973), have described use of glucose oxidase chemically conjugated to antibodies capable of binding to target cells, thereby targeting the cell killing activity against those cells which it is desired to eliminate selectively. Cell killing, however, was only achieved if other (non-targeted) enzymes were present in the surrounding medium to generate even more toxic species.

Okuda et al, Infection and Immunity 27 690 (1980), describe a development of the Knowles et al work. Okuda et al used two enzymes both chemically conjugated to antibody to target cells. The enzymes were xanthine oxidase, which at least predominantly produces superoxide rather than peroxide and lactoperoxidase. Over a period of 90 minutes, in vitro, they achieved a reduction of live Candida albicans cells, in vitro, which was between one and two orders of magnitude better than was achieved with lactoperoxidase in solution.

Arnold et al J. Dent. Res. 59B, 961 (1980) have described use of a glucose oxidase/antibody chemical conjugate against oral bacterial, but achieved only reversible bacteriostasis rather than cell killing.

Lal et al Journal of Immunological Methods 79 307 (1985) used enzyme chemicall conjugated to mouse anti-rat antibodies together with rat antibodies to target cells. They were able to kill a proportion of the target cells.

WO-89/11866 discloses a bactericide chemically conjugated to an antibody which binds to bacteria associated with dental plague/caries.

EP-A-361908, corresponding to WO-90/3185, both published in April 1990, and WO-90/112 published in January 1991 both disclose enzymes, with a cytotoxic action, attached through covalent bonding to an antibody which is able to bind to a target site.

Pene et al, Biochemistry International 13 233 1986 discloses enzymes chemically conjugated to goat anti-rabbit antibodies which bind to rabbit antibodies to target myeloma cells.

EP-A-315364 describes an immunoassay procedure which entails forming a complex of a first antibody or antigen, a second antibody or antigen and the antigen or antibody to be determined.

SUMMARY OF THE INVENTION

In the present invention an agent to be delivered to a target site is bound to the target site by use of a plurality of antibodies or antibody fragments to form a bridge between the agent and the target site. The complex which results has the ability to assemble itself.

Accordingly, in the present invention one or more vehicles contain in the same or separate vehicles,
i) a first antibody or antibody fragment to bind to a target site, and
ii) one or more further antibodies or antibody fragments able collectively to bind by means of antibody-antigen binding both to an active agent and to the first antibody or antibody fragment, thereby to attach the agent to the target site.

One aspect of the invention resides in a product comprising the above-mentioned vehicles. Another aspect is a method of delivering an active agent, especially enzyme(s) by administration of the vehicles.

The active agent may be included in one of the vehicles, but alternatively the agent may be a material which is present in vivo in the general vicinity of the target site. The antibodies or fragments thereof would then serve to concentrate the agent onto the target site, thereby enhancing its activity against the target.

By relying on antibody-antigen binding, active agent(s) can be coupled to a target site as complexes which assemble spontaneously when their constituent parts come together.

BRIEF DESCRIPTION OF THE DRAWINGS

Some arrangements embodying the invention are illustrated by way of example in the accompanying drawings, which are all schematic diagrams of target sites and coupled enzyme(s). In these diagrams G.Ox denotes glucose oxidase, HRP denotes horseradish peroxidase and PEI denotes polyethyleneimine.

In the drawings:-

Fig. 1 shows whole antibodies used to attach glucose oxidase to a target;

Fig. 2 shows similar attachment of a more complex therapeutic agent;

Fig. 3 shows attachment of glucose oxidase as in Fig. 1 plus chemical conjugation of a second enzyme;

Fig. 4 shows attachment of two enzymes to a target, where both are attached by whole antibodies; and

Fig. 5 shows attachment to enzymes to a target, where antibody fragments are used.

DETAILED DESCRIPTION OF EMBODIMENTS

As set forth above, a first antibody or antibody fragment binds to a target site and one or more further antibodies or fragments collectively attach an agent to the target site.

If the invention is implemented with whole antibodies, the said further antibodies may be an antibody to the active agent, derived from (e.g. generated in) the same species as the antibody to the target site, and another species' antibody to immunoglobulin of the first species, which will act as a bridge and enable spontaneous complex formation.

For instance, rabbit antibodies to S. mutans (as target) and to glucose oxidase (as therapeutic agent) can be used together with goat antibody to rabbit immunoglobulin. The goat antibody will have plural sites for binding rabbit immunoglobulin antigens, and hence the enzyme can be bound through a chain of antibody/antigen connections. This consists of:

target (S. mutans) bound to rabbit anti-S.mutans goat anti-rabbit immunoglobulin bound to the rabbit anti-S. mutans and also bound to rabbit anti-glucose oxidase which is bound to glucose oxidase itself.

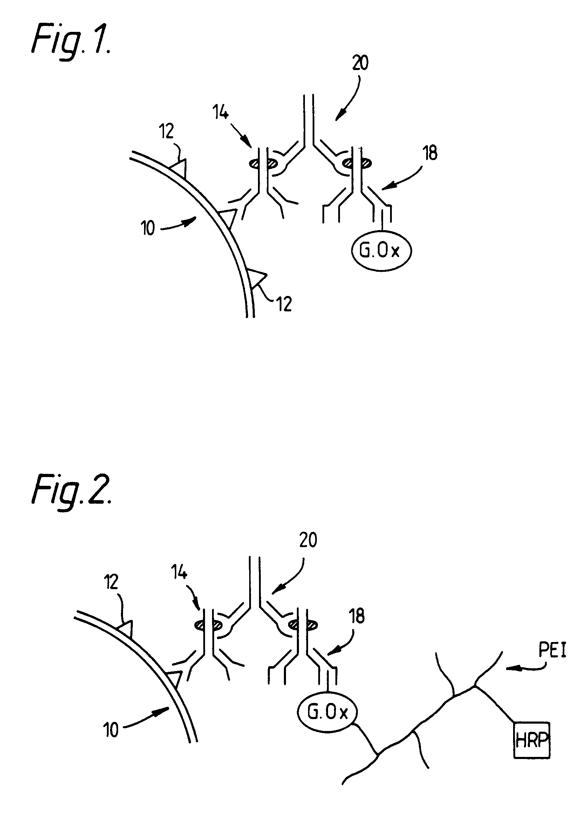

Such an arrangement is shown in Fig. 1, in which numeral 10 denotes a target, which is S. mutans, with antigenic sites 12. Numeral 14 denotes the first antibody which is rabbit anti-S. mutans. Numeral 18 denotes rabbit anti-glucose oxidase binding to that enzyme (G.Ox). Numeral 20 denotes the bridging antibody which is goat anti-rabbit. This arrangement serves to bind the cytotoxic enzyme to the chosen target site through a specific antibody to that site. We have demonstrated such binding in vitro, with killing of the target cells.

The invention can also be implemented using antibody fragments, notably utilising a first antibody fragment to bind to the target site, together with one or more further fragments for binding the active agent to the first fragment, thereby to attach the agent to the target site.

The first antibody fragment may be an Fv fragment of an antibody to the desired target. Such a fragment contains only the variable domains of light and heavy chains of an antibody. The fragment could possibly be an $F(ab)_2$ fragment which would provide two combining sites. It might alternatively be as little as a single variable domain of one chain of an antibody.

Techniques for efficient production of biologically active antibody fragments in E. Coli were described by A Skevia and A. Pluckthun (1988), Science, 240, 1038; M. Better et al, (1988), Science, 240, 1041; and E.S. Ward et al, (1989), Nature, 341, 544. The cloning and expression of genes encoding antibody fragments in E. Coli is also described in published European application EP-A-368684 (Medical Research Council).

We prefer that a first antibody fragment has an additional peptide chain appended to it, and further fragments bind the active agent to this peptide chain. Bacteria can be made to produce a variable domain with an extra peptide chain already attached to the C-terminus of the domain. This can be achieved by standard genetic techniques. For example, a gene encoding a variable domain can easily be lengthened by means of site directed mutagenesis techniques, using in vitro synthesised oligonucleotides which encode the peptide to be appended to the variable region. Site directed mutagenesis is now a widely used technique, and adequate protocols can be found in several published books, for example in Sambrook et al, (1989), Molecular Cloning, 2nd edition, Cold Spring Harbour Laboratory Press, New York. An attached extra peptide provides a very convenient "handle" for the attachment of the therapeutic agent.

Further antibody fragments which bind the active agent to the first fragment are preferably a second antibody fragment able to bind to the active agent by antibody-antigen binding, and an $F(ab)_2$ fragment (which is bivalent) able to bind to the first and second antibody fragments, especially to antigenic peptides appended to the first and second antibody fragments.

3

The present invention could be used to deliver to a variety of different target sites, especially target sites which are accessible by topical application to the body surface, target sites in the mouth, and target sites in material temporarily removed from the body. One significant application is delivery of cytotoxic agents to species of the supragingival oral microflora.

The oral microflora is a complex ecosystem which contains a wide variety of microbial species. One of these species may be selected as the target site. However, the effect of targeting to one species will be to attack both that species and other species which occur in close proximity to it. Thus, by delivering to one species which occurs in dental plaque, cytotoxic agents will be delivered to the plaque and will act against all the species which occur together in the plaque, including those responsible for plaque formation.

Extra-cellular dextran produced by such organisms could itself be used as the target site in which case the first antibody or antibody fragment has binding affinity for dextran.

One possible target species is Streptococcus mutans. This has been identified as an important contributor to dental plaque, and has been shown to be capable of inducing clinical caries lesions in germ-free animals when established as a mono-infection. S. mutans has the ability to utilise dietary carbohydrate for the synthesis of an insoluble polysaccharide matrix, facilitating attachment to, and colonisation of, hard surfaces, as well as production of acids capable of the dissolution of enamel. These characteristics have been identified as important virulence determinants. Although other species and genera have also proved capable of both acid and plaque production, or even or caries initiation in the germ-free animal, S. mutans is widely recognised as at least one significant cause of tooth decay because of the scale of its acid and polysaccharide production.

Other species which may be selected as the target species are S. sanguis, A. viscosus and A. naeslundii. These are all present in dental plaque as a substantial proportion of the species normally found in dental plaque. Because of frequent occurrence these three may be preferred target species.

Consequently, in a particularly envisaged application of this invention, the first antibody is an antibody to S. mutans, S. sanguis, A. viscosus or A. naeslundii and the active agent is the enzyme glucose oxidase.

Another application is to attack species of the subgingival microflora responsible for periodontal disease. The target species could well be Bacterioides gingivalis.

For any oral application (dental care) it would be necessary for the vehicle(s) in the product to be acceptable to enter the mouth, e.g. vehicle(s) suitable for topical application in the mouth.

Another possible application is to deliver therapeutic agents to attack human tumour cells, notably in bone marrow which has been removed temporarily from the body of a patient undergoing radiotherapy.

Various materials are contemplated as the active agents which may be delivered in accordance with this invention. One important possibility is an enzyme able to generate a cytotoxic product: glucose oxidase has been mentioned above. Galactose oxidase is another. If the target site is in the mouth, the substrate for this enzyme could be the galactose which occurs naturally in yoghurt. Further possibilities are xanthine oxidase which produces superoxide and NADP oxidase which also does so.

Other enzymes which might be delivered to a target site are lysozyme, to attack the cell wall of a gram negative bacterium or proteases to attack extracellular enzymes such as those which help to form dental plaque.

Another category of materials which could be delivered by means of the invention are enzyme inhibitors, which could act to inhibit extracellular enzymes of a target organism.

Further possibilities are oxygen generators or acid producers which could act to modify the environment in the vicinity of a target site. A particular possibility arises if the target site is in the periodontal pocket. Generating oxygen or reducing pH in this cavity would give conditions less favourable for the anaerobic organisms which can invade this pocket.

Yet another possibility is to deliver a material able to remove a limiting nutrient. More specifically, aerobactin, enterochelin or porphyrin could be delivered to a target in the periodontal pocket. They would complex with iron and reduce the concentration of iron available to bacterial invaders of this pocket. These materials could be used as such as the therapeutic agent. However, they could be used in glycosylated form in which the glycosyl chains would be antigenic, for an antibody or fragment to bind to the glycosyl chain. Alternatively they could be covalently bound to some other moiety to which an antibody could attach.

Another possible type of active agent is a non-enzymic catalyst. This could be a transition metal attached to ligand(s). For instance Borggaard, Farver and Andersen, Acta Chem. Scand. 25 [1971] 3541 have shown that iron with ethylene diamine tetraacetic acid (EDTA) as ligand will catalyse the conversion of hydrogen perioxide to hydroxyl ion and an OH free radical which would be very short lived but also very toxic.

A possible cosmetic application is the reduction of stain on teeth. In this application the active agent is an enzyme which produces a bleaching substance such as hydrogen peroxide. The target site is at the tooth surface. Binding to the target in accordance with this invention serves to locate the enzyme adjacent to the surface. In consequence a bleaching substance is produced in the vicinity of the tooth surface which may be stained.

Within the scope of this invention, the active agent and antibodies or fragments of antibodies could be contained in one vehicle or distributed among a plurality of vehicles. In the former case the complex of active agent and antibodies will form in the vehicle. In the latter case, parts of the complex may link together if they are capable of doing so and are in the same vehicle. However, formation of the full complex can only take place when all of its components come together.

If polyclonal antibodies are being used, we have found that the active agent and antibodies should be distributed between at least two vehicles, so as to avoid full complex formation in a vehicle during storage. We have found that during storage full complexes formed with polyclonal antibodies suffer a reduction in their ability to bind to the target site. Partial complex formation is tolerable: for instance linking of enzyme to an anti-enzyme antibody within a single vehicle is acceptable since the resulting conjugates appear to be storage stable. If the first (anti-target) antibody does not bind directly to the antibody which binds to the therapeutic agent, these two antibodies may both be included in the same vehicle. Then a second vehicle can contain the antibody which will enable fuel complex formation.

One possibility is that the first and further antibodies are all whole antibodies and at least one of them is monoclonal.

Distribution of constituents of the complex between a plurality of vehicles may be unnecessary if monoclonal antibodies are employed. In general, monoclonal antibodies would form smaller complexes and during storage would be expected to retain their activity better than complexes formed with polyclonal antibodies. This is also true when antibody fragments are used. An advantage of antibody fragments is that the complexes which form by antigen - antibody binding are fairly small and more stable than complexes with whole antibodies.

A product comprising a vehicle or vehicles containing therapeutic agent and antibodies or antibody fragments could take a number of forms. If the target site is in the mouth, possibilities are mouthwash, toothpaste and a lozenge which will dissolve in the mouth. These forms of product could be used even when a plurality of vehicles are needed. For instance the product could be a two-component mouthwash which the user mixes immediately before use.

It could be a toothpaste having two components stored in the toothpaste container in such a way that they are kept separate or at least do not mix but are dispensed together and mix in the mouth of the user. Such two-component toothpaste products are known per se.

Another possible form of product providing a plurality of vehicles would be a lozenge to be sucked in the mouth, with the various components of the complex contained in separate regions of the lozenge. Three separate regions would preferably be used, and arranged so that the components of the complex were released in order, starting with the first (anti-target) antibodies. This would allow the complex to assemble on the target site.

Two vehicle forms could be used in combination as a way to provide a plurality of vehicles, e.g. toothpaste whose use is followed by a mouthwash or a lozenge.

When the active agent is an enzyme, the product may include a substrate for enzyme action, or it may rely on the enzyme substrate being present at the target site. Thus where the enzyme is glucose oxidase, directed at a target site in the mouth, the product could rely on dietary glucose as the enzyme substrate or it could itself incorporate gluclose provided this was kept separate from the glucose oxidase.

A development of the invention is to utilise more than one enzyme able to co-operate together, with antibodies or antibody fragments serving to bind both enzymes to the target site. Thus the enzymes will be held in proximity to each other and to the target.

This development can be implemented in several ways. One possibility would be for the active agent to comprise the two enzymes both attracted to a common intermediary.

If both enzymes are attached to a common intermediary, this may be a synthetic polymer having chemical functionality to enable the attachment of enzymes by chemical reaction. A suitable polymer is polyethyleneimine (PEI) which is a branched polymer with amino groups at the ends of the branches. An arrangement illustrating this is shown as Fig. 2 where numerals 10 to 20 have the same significance as in Fig. 1. The enzymes are glucose oxidase (G.Ox) and horseradish peroxidase (HRP).

Glucose oxidase and horseradish peroxidase are both enzymes with pendant glycosyl chains. Such enzymes can be covalently bound to polyethyleneimine by first oxidising the enzymes in aqueous solution with periodate to generate aldehyde groups in the pendant glycosyl chains. These groups will then form Schiff bases with amino groups on the polyethyleneimine, at alkaline pH (e.g. pH 9.5) after which reduction with borohydride can be used to reduce any unreacted aldehyde groups and also increase stability by reduction of the Schiff bases.

Another possibility for utilising two enzymes is that one enzyme is attached to the target site by antibody-antigen binding in accordance with this invention, while the other enzyme is chemically conjugated (i.e. by covalent bond formation) to one antibody involved in binding the other enzyme.

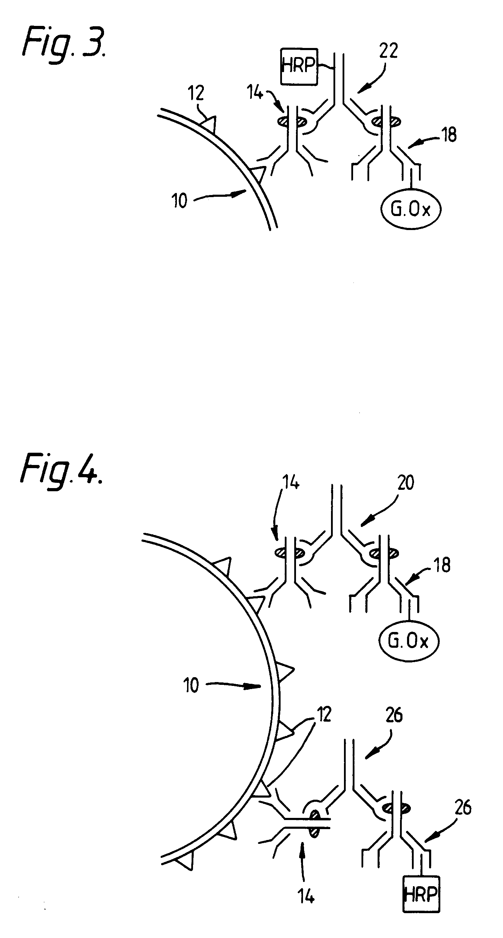

Chemical conjugation of one enzyme to an antibody may be performed by the above technique of Schiff base formation. In this case, the enzyme could be conjugated to a bridging antibody which in use binds to anti-target antibody and to an antibody to the other enzyme. This is illustrated by Fig. 3, where reference numerals have the same meaning as before. Horseradish peroxidase (HRP) is chemically conjugated to goat anti-rabbit immunoglobulin 22,20.

A third possibility for utilising two enzymes is that both enzymes are separately attached to the target through antibody-antigen binding in accordance with this invention. A separate antibody or antibody fragment will generally be required to bind to each of the two enzymes.

A single first antibody or fragment may be used and if a bridging antibody or fragment is used to connect the first (anti-target) antibody or fragment with the respective anti-enzyme antibodies or fragments, a single bridging antibody or fragment able to bind to both anti-enzyme antibodies or fragments is preferred. Conceivably, though, separate systems with different first antibodies or fragments and bridging antibodies or fragments could be used in admixture, both binding to the same target site.

Of special interest is the combination of an oxidase such as glucose or galactose oxidase and a peroxidase such as horseradish peroxidase which uses peroxide to convert halide ions to oxidised halide species that are even more toxic than peroxide. A peroxidase may also use peroxide to convert thiocyanate to hypothiocyanate. Oxidised halide or hypothiocyanate species may also be generated for the purpose of bleaching tooth stain.

Where whole antibodies are used, it is preferred that antibodies to each of the two enzymes come from the same species as an antibody to the target site. Another species' antibody to that species will act as an indiscriminate bridging antibody, forming complexes to link both of the enzymes to the target site. This arrangement is illustrated by Fig. 4 where reference numerals have the same meaning as before. Numeral 26 denotes rabbit-anti horseradish peroxidase.

If two enzymes are employed, the product may include substrates for both of them e.g. both glucose and a halide. Alternatively, only one substrate, or none at all may be included and reliance placed on substrate which comes from another source and happens to be present at the target site.

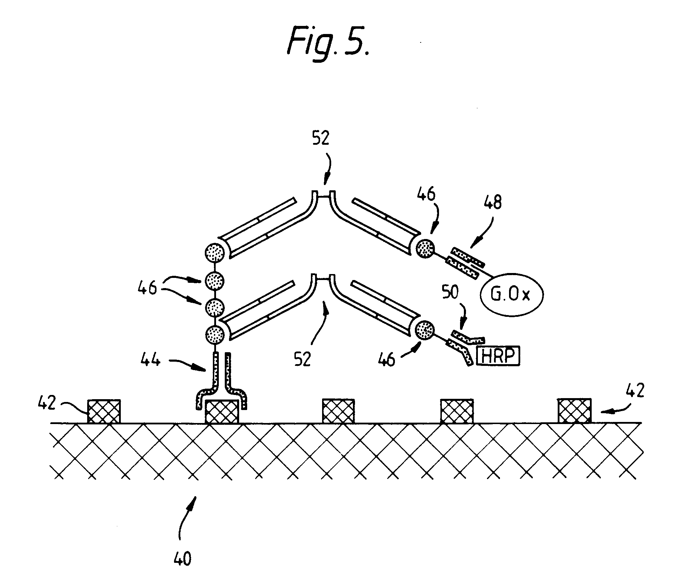

Fig. 5 shows a preferred arrangement where antibody fragments are utilised.

For attaching to the target 40 which has antigenic sites 42 there is an Fv antibody fragment 44 with several repeats of a peptide 46 appended to the distal (C-terminal) end of one of the two chains in the Fv fragment 44.

The active agents are glucose oxidase 16 and horseradish peroxidase 24. Each is bound by a respective Fv fragment 48, 50 with specificity for the enzyme concerned and with a single repeat of the same peptide 46 appended to one chain of the Fv fragment.

The peptides 46 appended to the anti-enzyme Fv fragments become linked to peptides 46 on the anti-target Fv fragment by $F(ab)_2$ fragments 52 which bind specifically to these peptides. Since the $F(ab)_2$ fragments are divalent they can form a bridge attaching an anti-enzyme fragment, with attached enzyme, to the anti-target fragment 44.

EXAMPLES

The invention is further explained by the following experimental Examples, demonstrating effectiveness of the system in vitro

In the Examples:

"PBS" denotes phosphate buffered saline having pH 6.5.

Glucose oxidase was commercial enzyme derived from Aspergillus niger (Sigma Type VII).

Goat anti rabbit Ig antibodies were commercially available from Atlantic Antibodies.

Dilutions are expressed in the form "1/n" signifying that a quantity of starting solution was mixed with diluent to give a solution in which the concentration was one n'th that of the starting solution.

## Example 1

### Setting up a Glucose Oxidase/anti-Glucose Oxidase Complex

This Example describes a technique which can be used to give an approximate indication of suitable working concentrations of components of such a complex, suitable as a starting point. Later examples show that variations of concentrations are possible, nonetheless.

The complex was to be formed using glucose oxidase enzyme and three antibodies which were rabbit anti-glucose oxidase, goat anti-rabbit Ig, and rabbit anti-S. mutans. Working concentrations for the enzyme and the first two antibodies were established using an ELISA format with a solid phase consisting of immobilised rabbit IgG to mimic rabbit antibodies attached to S. mutans. This was carried out as follows.

Nylon pegs were washed with ethanol, then exposed successively to:

Glutaraldehyde (aqueous, 2%) as coupling agent Rabbit IgG solution (50μg protein per ml in PBS) Bovine serum albumin (0.2%, in PBS) to block excess coupling agent.

Between and after these exposures the pegs were washed with PBS. After coupling to rabbit IgG in this way the pegs were fitted into purpose made holders from which they projected. These were subsequently used to support the pegs projecting into wells of microtitre trays.

Three trial solutions of glucose oxidase in PBS were made up, with concentrations of 40, 20 and 10μg/ml.

Four trial solutions of a commercially available goat anti-rabbit Ig in PBS were prepared by diluting a stock solution to 1/125, 1/250, 1/500 and 1/1000 of its original concentration.

Rabbit anti-glucose oxidase serum was prepared by immunising a rabbit intra muscularly with purified glucose oxidase from Aspergillus niger (Sigma type VIII) as a water in oil in water emulsion in Freunds complete adjuvant followed by 2 booster inoculations in Freunds incomplete adjuvant. The presence of specific anti-glucose oxidase antibodies in serum from the rabbit was demonstrated by standard immunological double diffusion procedures, and by solid phase immunosorbent capture in polystyrene microtitre trays.

Four trial solutions containing the rabbit anti-glucose oxidase antibodies were made from the immunoglobulin fraction of the immunised rabbit serum with concentrations of 20, 10, 5 and 2.5μg of rabbit IgG/ml. (These were obtained by dilution of a stock solution containing 13mg of rabbit IgG per ml).

Complexes were formed in wells of a standard 96 well microtitre tray by mixing equal volumes of trial solutions respectively containing glucose oxidase, goat anti-rabbit Ig, and rabbit anti-glucose oxidase. The trial solutions were mixed in 48 combinations. The mixtures were incubated at room temperature for 45 minutes, after which a nylon peg, previously sensitized with rabbit IgG as described above, was inserted into each well and incubated for 1 hour at room temperature.

The quantity of complex binding to each peg was assayed by removing the peg, washing it and placing it in a substrate mixture consisting of tetramethyl benzidine (TMB 100μg/ml), horseradish peroxidase (10μg/ml) and glucose (150mM) in phosphate/citrate buffer, pH 6.5. Colour was allowed to develop for 30 minutes at room temperature, and the optical density measured.

For each dilution of goat anti-rabbit IgG, optical density was plotted against the concentration of rabbit anti-glucose oxidase. It was apparent that the best dilution of the goat anti-rabbit IgG reagent was 1/250 and, at this dilution, the rabbit anti-glucose oxidase should be used at the 20μg/ml concentration. 40μg/ml of glucose oxidase performed best although the concentration of glucose oxidase made very little difference to the bound activity, at least between 10 and 400μg/ml. Therefore, any of these glucose oxidase concentrations could be adopted.

## Example 2

### Bactericidal Activity of Complex

An experimental procedure was carried out in which S. mutans was exposed to rabbit anti-S. mutans, then a complex which was similar to that described in the previous Example and then glucose which should serve as substrate for glucose oxidase bound by the complex to the S. mutans cells, leading to killing of the cells. Controls were carried out in which steps of the procedure were omitted.

The procedure was a series of steps as follows:

1. 10ml aliquots of a culture of S. mutans in Todd Hewitt broth were transferred to sterile McCartney bottles. The bacterial were centrifuged into a pellet, washed 3 times with PBS and resuspended into 9ml PBS.

2. 1ml of rabbit anti-S.mutans suspension was added. This suspension was a 1/40 dilution in PBS of rabbit anti-S. mutans serum obtained by the method of Edwards and Ewing described in "Identification of Enterobacteriaceae", Burgess Publishing Co, Minneapolis, 1955. The dilution reached on addition to the McCartney bottle was of course 1/400. Alternatively 1ml of PBS was added as control.

3. The suspension was incubated at ambient temperature for 1 hour, then the cells were centrifuged into a pellet and washed three times with PBS and resuspended in 9ml PBS as before.

4. 1ml of glucose oxidase/antibody complex was added. This complex was previously prepared by mixing equal volumes of glucose oxidase (500μg/ml), rabbit anti-glucose oxidase at 1/200 dilution in PBS and goat anti-rabbit IgG at 1/100 dilution in PBS and then allowing complex formation during 1 hour at ambient temperature. All of these were the same materials as for the previous Example but at higher concentration to take subsequent dilution into account. As a control 1ml of PBS was added in place of the complex.

5. Again the suspension was incubated at ambient temperature for 1 hour, then the cells were centrifuged into a pellet and washed three times with PBS.

6. The cells were resuspended in 10ml of 0.5% w/v filter sterilised D-glucose. In a control, PBS was used, and for a comparison 0.5% w/v filter sterilised L-arabinose was used in place of glucose.

7. The resulting suspension was incubated at ambient temperature for 24 hours and 0.5ml samples were taken immediately on mixing, and at intervals. The bacteria in each sample were separated by centrifugation into a pellet which was washed three times with PBS. The viable cells in each sample were assayed by the method of Miles, Misra and Irwin J. Hygiene 38, 732-749, (1938). The combinations of materials added, and the counts of viable cells are set out in the following Table 1.

TABLE 1

NUMBERS OF SURVIVING CELLS

| ADDITIONS | | | Duration of Incubation in Substrate (hours) | | | | | | |
|---|---|---|---|---|---|---|---|---|---|
| Step 2 | Step 4 | Step 6 | 0 | 1 | 2 | 3 | 4 | 7 | 24 |
| Ab* | PBS | PBS | $1.6 \times 10^6$ | $1.38 \times 10^6$ | $1.18 \times 10^6$ | $1.24 \times 10^6$ | $4 \times 10^5$ | $4.6 \times 10^4$ | $1 \times 10^5$ |
| Ab | Cplx | Glucose | $1 \times 10^6$ | $1 \times 10^6$ | $3 \times 10^5$ | $2.8 \times 10^5$ | $1.6 \times 10^5$ | $6.8 \times 10^4$ | 0 |
| PBS | Cplx | Glucose | $1.4 \times 10^6$ | $8.2 \times 10^5$ | $4 \times 10^5$ | $3.2 \times 10^5$ | $2.2 \times 10^5$ | $1 \times 10^5$ | $5.8 \times 10^3$ |
| Ab | Cplx | Arabinose | $2.6 \times 10^6$ | $1.32 \times 10^6$ | $6 \times 10^5$ | $1.02 \times 10^6$ | $6 \times 10^5$ | $5.8 \times 10^4$ | $2 \times 10^5$ |
| PBS | Cplx | Arabinose | $8 \times 10^5$ | $7.8 \times 10^5$ | $4 \times 10^5$ | $8 \times 10^5$ | $3.4 \times 10^5$ | $3.2 \times 10^5$ | $8 \times 10^4$ |

* Ab denotes rabbit anti-S. mutans as described for step 2.

It can be seen from Table 1 that use of the complex and glucose achieved cell killing which did not take place when the rabbit anti-S. mutans antibodies were used without the complex. This demonstrates that glucose oxidase was being attached to cells via the complex, because very little free glucose oxidase or even unbound complex would have been carried through the washing steps.

Surprisingly there was some killing even without the rabbit anti-S. mutans antibodies, suggesting a low level of binding of the complex directly to S. mutans.

Example 3

As a further demonstration that the complex will bind glucose oxidase to cells, Example 2 was repeated as far as its step 5, after which the pellets of cells were washed three times by repeated centrifugation and resuspension in a phosphate/citrate buffer. The washed cells were resuspended in a solution of glucose (150mM), horseradish peroxidase (10μg/M1), and tetramethylbenzidine (100μg/ml) in PBS. The reaction mixture was formed in a microtitre well. It was incubated at ambient temperature for 30 minutes and then the optical density was measured and compared with a calibration curve obtained using standard solutions of glucose oxidase.

The additions to the S. mutans cells, and the bound glucose oxidase levels determined coloimetrically are given in Table 2 below.

TABLE 2

| ADDITIONS | | GLUCOSE OXIDASE LEVEL |
|---|---|---|
| Step 2 | Step 4 | |
| Ab | PBS | Nil |
| PBS | Complex | 13ng/ml |
| Ab | Complex | 185ng/ml |

Example 4

Example 2 was repeated, using different dilutions of serum at step 2, and different concentrations of complex constituents.

For step 2, three different dilutions of the anti-S. mutans serum were used. These were 1/4, 1/10 and 1/40 dilutions in PBS, giving final dilutions, when added to the McCartney bottles, of 1/40, 1/100, and 1/400. PBS was also used as a control at step 2.

Two complexes were made and used at step 4. The concentrations used to make the complexes were:

| | Complex I | Complex II |
|---|---|---|
| Goat anti-rabbit IgG | 1/100 dilution | 1/10 dilution |
| Rabbit anti-glucose oxidase | 1/200 dilution | 1/100 dilution |
| Glucose oxidase | 500μg/ml | 200μg/m. |

Complex 1 was thus as used in Example 2 whereas Complex II was as indicated by Example 1 (bearing in mind that the complex is diluted tenfold when added to the McCartney bottle).

For step 6 there was used 0.5% w/v filter sterilised glucose solution as in Example 2, and viable cell counts were determined as in Example 2. The various additions and results are set out in the following table.

10

TABLE 3

NUMBERS OF SURVIVING CELLS PER ML

| Final Dilution of antiserum Step 2 | Complex used Step 4 | Duration of Incubation in Substrate (hours) | | | | | | |
|---|---|---|---|---|---|---|---|---|
| | | 0 | 1 | 2 | 3 | 4 | 8 | 24 |
| 1/40 | PBS | $5 \times 10^5$ | $2.2 \times 10^5$ | $3.2 \times 10^4$ | $8 \times 10^4$ | $7.2 \times 10^4$ | $8.8 \times 10^5$ | $5.6 \times 10^5$ |
| PBS | Cplx I | $1.2 \times 10^5$ | $8.6 \times 10^4$ | $6.6 \times 10^4$ | $4.6 \times 10^4$ | $2 \times 10^4$ | $2 \times 10^4$ | $3.2 \times 10^2$ |
| 1/40 | Cplx I | $1.6 \times 10^5$ | $7.2 \times 10^4$ | $4 \times 10^4$ | $3.2 \times 10^4$ | $1.2 \times 10^4$ | $3.0 \times 10^3$ | 0 |
| 1/100 | Cplx I | $1.4 \times 10^5$ | $7.8 \times 10^4$ | $4 \times 10^4$ | $2.4 \times 10^4$ | $8 \times 10^3$ | $3.2 \times 10^3$ | 0 |
| 1/400 | Cplx I | $1.8 \times 10^5$ | $6 \times 10^4$ | $3.4 \times 10^4$ | $1.8 \times 10^4$ | $8.8 \times 10^3$ | $4.4 \times 10^3$ | 0 |
| PBS | Cplx II | $4.4 \times 10^5$ | $4 \times 10^5$ | $2 \times 10^5$ | $4 \times 10^5$ | $3.6 \times 10^5$ | $2.6 \times 10^5$ | $1.04 \times 10^4$ |
| 1/40 | Cplx II | $1.24 \times 10^6$ | $2.6 \times 10^5$ | $5 \times 10^5$ | $1.6 \times 10^5$ | $2 \times 10^5$ | $7.6 \times 10^3$ | 0 |
| 1/100 | Cplx II | $1.24 \times 10^5$ | $2.6 \times 10^5$ | $4.4 \times 10^5$ | $2 \times 10^5$ | $1.4 \times 10^5$ | $1 \times 10^4$ | 100 |
| 1/400 | Cplx II | $6 \times 10^5$ | $3.6 \times 10^5$ | $1.8 \times 10^5$ | $1.6 \times 10^5$ | $3.8 \times 10^5$ | $1 \times 10^5$ | $9.7 \times 10^2$ |

As can be seen from Table 3, with Complex I variations in the level of the targeting antirserum had no effect on the efficiency of cell killing, with all dilutions giving a 100% kill after 24 hours, and no real difference between them at any time before that. Complex I without targeting antiserum had some cytotoxic effect (due to non-specific or cross-reactive binding), but this was not as potent as the complete complex and did not lead to 100% kill.

11

Complex II also gave a 100% kill after 24 hours but only with 1/40 diluted targeting anti-serum.

Example 5

This Example demonstrates the killing of human tumor cells (cultured LoVo cells) using glucose oxidase and peroxidase, both targeted to the cells.

LoVo cells were detached from a tissue culture and suspended in RPMI 1640 tissue culture medium. Aliquots of the suspension were pipetted into wells of a tissue culture plate which was incubated for 4 days at 37°C for LoVo cells to attach to the plate. The culture medium was tipped off the culture plate and an aliquot of mouse anti-LoVo antibody suspension was added to each well. This antibody was a monoclonal antibody, designated AUA-1 (ex Unipath Ltd.) Each aliquot was 200µl of a 1/1000 dilution of AUA-1 antibody suspension).

The plate was incubated for 1 hour at 37°C, then the antibody suspension was tipped off. Each well next received a 200µl aliquot of a solution containing the following:

| glucose oxidase (g.Ox) | 10µg/ml |
| horseradish peroxidase (HRP) | 10µg/ml |
| mouse anti-G.Ox antibody | 1250ng/ml |
| mouse anti-HRP antibody | 250ng/ml |
| rabbit anti-mouse antibody | 50µg/ml |

It would be expected that two types of complex would form by antibody-antigen binding, viz:
a) LoVo:Mouse anti-LoVo:Rabbit anti-mouse:Mouse anti-G.Ox:G.Ox
b) Lovo:Mouse anti-LoVo:Rabbit anti-mouse:Mouse anti-HRP:HRP

This is the arrangement illustrated by Fig. 4, with LoVo cells as the target.

Some wells were used as controls, and received an aliquot of a solution containing only the enzymes.

Every well next received a 50µl portion of a catalase solution. Various catalase concentrations were used. The catalase functioned to destroy hydrogen peroxide.

The plate was incubated for 1 hour at 37°C after which the extent to which the LoVo cells remained viable was determined. To do this 100µl of solution was removed from each well and replaced by 50µl of a 1mg/ml solution of: (3-(4,5 - dimethylthiazole-2-yl) 2,5 diphenyl) tetrazolium bromide (MTT) (Sigma Chemical Co). Living cells generated blue crystals of formazan. These blue crystals were dissolved by addition of 100 µl of 0.04 N HCl in isopropanol to each well. The optical density was read on an ELISA plate reader at 570 nm. The results were all either a fairly low absorbance, approximately 0.2, denoting an absence of live cells, or a fairly high absorbance approximately 1.1 if live cells were present.

The results were:

| Catalase concentration (µg/ml) | Absorbance at 570nm | |
| --- | --- | --- |
| | Enzyme solution | Enzymes and antibodies |
| 0 | L | L |
| 2.5 | H | L |
| 5 | H | L |
| 10 | H | L |
| 20 | H | L |
| 40 | H | H |
| 80 | H | H |
| L = 0.2 within experimental error | | |
| H = 1.1 within experimental error | | |

Thus, without catalase present, even the free enzymes killed the LoVo cells. At 40µg/ml or more of catalase, its scavenging of $H_2O_2$ suppressed all cell killing. Lower concentrations of catalase suppressed cell killing by free enzymes but not when the antibodies were present. This demonstrates that the enzymes were able to kill cells and that the antibodies were making the enzymes more effective by attaching them to the target cells.

Example 6

Like Example 5, this example demonstrates the killing of cells using glucose oxidase and a peroxidase, both targeted to the cells. The cells in this example were S. sanguis which is a species that occurs in the mouth. The antibodies used in this example were made by standard techniques.

S. sanguis cells were centrifuged out of a culture broth, washed three times by resuspending in PBS and centrifuging, then resuspended in PBS. Aliquots were placed in each of a number of tubes, centrifuged, and resuspended in a filter sterilized suspension (in PBS) of mouse polyclonal anti-S. sanguis antibodies (obtained from ascites fluid). The suspensions were incubated at room temperature for 30 minutes to allow the antibodies to bind to the cells. The cells were again centrifuged and washed with PBS.

Some tubes, used as a comparison, received PBS in place of the mouse anti-S. sanguis suspension.

Next the cells were resuspended in 1ml of PBS containing

| glucose oxidase (G.Ox) | 100$\mu$g/ml |
| horseradish peroxidase (HRP) | 100$\mu$g/ml |
| mouse anti-C.Ox monoclonal antibody | 1.25$\mu$g/ml |
| mouse anti-HRP monoclonal antibody | 1.25$\mu$g/ml |
| rabbit anti-mouse polyclonal antibody | 50$\mu$g/ml |

(The rabbit anti-mouse antibody was added to a solution already containing the other four materials).

In some tubes PBS alone was substituted for the above solution containing enzymes and antibodies. In further tubes a solution containing enzymes and antibodies was used but rabbit anti-mouse antibody was omitted.

All of the suspensions were incubated at room temperature for 30 minutes to allow the enzymes to become bound to the cells. Then the cells were centrifuged out, washed with PBS and resuspended in 1ml of PBS containing 15$\mu$g/ml potassium iodide and 5%w/v glucose.

These suspensions were incubated for 24 hours at room temperature. Sample portions were removed from each suspension at the beginning of this incubation period and after periods of 1,2,3,4 and 24 hours. The viable cells in each sample were assayed in the same way as in Example 1 and the results are set out in the following Table 3.

TABLE 3

SURVIVING CELLS PER ML

| | Anti-target | Enzymes and antibodies | Duration of Incubation (hours) | | | | | |
|---|---|---|---|---|---|---|---|---|
| | | | 0 | 1 | 2 | 3 | 4 | 24 |
| A | Mouse anti-S. sanguis | None | $5 \times 10^7$ | $1.4 \times 10^7$ | $4.2 \times 10^7$ | $2.2 \times 10^6$ | $4 \times 10^7$ | $4.6 \times 10^5$ |
| B | Mouse anti-S. sanguis | All | $4.4 \times 10^7$ | $4 \times 10^6$ | $2.4 \times 10^7$ | $4.6 \times 10^5$ | $1.6 \times 10^5$ | $1$ |
| C | Mouse anti-S. sanguis | Rabbit anti-mouse omitted | $5 \times 10^7$ | $6 \times 10^6$ | $3.6 \times 10^7$ | $7.4 \times 10^6$ | $2.6 \times 10^7$ | $2.4 \times 10^4$ |
| D | None | All | $5.2 \times 10^7$ | $6.4 \times 10^6$ | $2.6 \times 10^7$ | $2.8 \times 10^6$ | $8 \times 10^6$ | $4.6 \times 10^4$ |

Sample B, with both enzymes attached to the target cells gave much more effective cell killing than in the other samples.

14

EP 0 453 097 B1

Example 7

This example provides an in vitro model of the use of targeted enzymes to bleach tooth stain. A nitrocellulose membrane was used as the model surface.

The membrane was incubated with normal mouse serum for one hour. Such serum contains mouse immunoglobulin. Consequently mouse antibodies became attached to the membrane. The membrane was then incubated with a 3% solution of bovine serum albumin to block any remaining binding sites on the membrane. Samples of membrane treated as above were used as control and test samples (4 of each).

Next the test samples of the membrane (but not the control samples) were incubated with a solution containing:

| glucose oxidase (G.Ox) | 100$\mu$g/ml |
| horseradish peroxidase (HRP) | 100$\mu$g/ml |
| mouse anti-G.Ox monoclonal antibody | 1.25$\mu$g/ml |
| mouse anti-HRP monoclonal antibody | 1.25$\mu$g/ml |
| rabbit anti-mouse polyclonal antibody | 50$\mu$g/ml |

similar to the solution used in the previous Example.

This led to formation of complexes as illustrated in Fig. 4, with nitrocellulose membrane providing the target.

The test samples and the control samples were next stained with tea. An infusion of leaf tea (2% by weight) in boiling water was left to cool to room temperature and incubated with the test and control samples for 5 minutes, after which the samples were rinsed in PBS for 1 hour to remove unbound colour.

The samples were incubated in a PBS solution of pH 7.2 containing 5.4% by weight glucose overnight (18 hours). It was expected that this would provide substrate for the bound enzymes, leading to production of reactive species in the vicinity of the tea stain.

The intensity of the stain of the samples was assessed before and after incubation with the glucose solution, using a Minolta CR 200 chromometer to assess reflectance. The result is expressed on a numerical scale.

The observed increases in reflectance, and the standard deviations were:

| Test samples (with enzymes) | 1.17 ± 0.042 |
| Control samples | 0.15 ± 0.31 |

This shows a statistically significant ($p < 0.05$) increase in brightness of the test samples, compared to the control samples.

**Claims**

1. A product comprising one or more vehicles which contain, in the same or separate vehicles:
   i) a first antibody or antibody fragment to bind by antibody-antigen binding to a target site,
   ii) one or more further antibodies or antibody fragments able collectively to bind by means of antibody-antigen binding both to an active agent and to the first antibody or fragment, thereby to attach the agent to the target site.

2. A product according to claim 1 which also includes the active agent.

3. A product according to claim 1 or claim 2 wherein the further antibodies or fragments are an antibody or antibody fragment which binds to the active agent and a third antibody or fragment able to bind both to the said first antibody or fragment and to the antibody or fragment which binds to the active agent.

4. A product according to claim 3 wherein the said first and further antibodies are whole antibodies and wherein the antibody to the agent and the first antibody are both derived from the same species and the third antibody is another species' antibody to that species.

5. A product according to any one of the preceding claims wherein the active agent is an enzyme.

15

6. A product according to claim 5 wherein the enzyme is an oxidase.

7. A product according to any one of the preceding claims wherein the said vehicle(s) contain two enzymes.

8. A product according to claim 7 wherein the further antibodies or antibody fragments comprise respective antibodies or fragments binding to each of the two enzymes and third antibody able to bind either of the anti-enzyme antibodies or fragments to the said first antibody or fragment.

9. A product according to claim 7 or claim 8 wherein the enzymes are an oxidase which generates peroxide and a peroxidase.

10. A product acccording to any one of the preceding claims wherein said first and further antibodies are whole antibodies and at least one of the antibodies is monoclonal.

11. A product according to any one of the preceding claims wherein the vehicle(s) is/are suitable for topical application in the mouth.

12. A product according to claim 11 wherein the first antibody or antibody fragment is an antibody to S. mutans, S. sanguis, A. viscosus or A. naeslundii.

13. A product according to any one of the preceding claims wherein the product is, or at least one vehicle is provided by, a mouthwash, a toothpaste, or a lozenge.

14. A method of delivering a cosmetically active agent to a target site comprising exposing the target site to a product according to any one of the preceding claims.

15. A method of delivering a cosmetically active agent to a target site which comprises exposing the site to one or more vehicles which contain, in the same or separate vehicles:
    i) a first antibody or antibody fragment to bind by antibody-antigen binding to a target site,
    ii) one or more further antibodies or antibody fragments able collectively to bind by means of antibody-antigen binding both to an active agent and to the first antibody or fragment, thereby to attach the agent to the target site.

16. Use of at least one antibody or fragment as specified in any one of claims 1 to 13 to prepare a product for topical application in order to attach an active agent to a target site.

**Patentansprüche**

1. Produkt, umfassend ein oder mehrere Vehicula, enthaltend in denselben oder getrennten Vehicula:
    i) einen ersten Antikörper oder ein erstes Antikörperfragment zur Bindung durch Antikörper-Antigenbindung an einen Zielort,
    ii) einen oder mehrere weitere Antikörper oder Antikörperfragmente, die in der Lage sind, gemeinsam durch Antikörper-Antigenbindung sowohl an einen Wirkstoff, als auch an den/das erste(n) Antikörper oder -fragment zu binden, wodurch der Stoff an den Zielort gebunden wird.

2. Produkt nach Anspruch 1, das ebenfalls einen Wirkstoff einschließt.

3. Produkt nach Anspruch 1 oder Anspruch 2, wobei die weiteren Antikörper oder -fragmente einen Antikörper oder ein Antikörperfragment darstellen, der/das an den Wirkstoff und einen/ein dritten/drittes Antikörper oder -fragment bindet, welche in der Lage sind, sowohl an den/das erste(n) Antikörper oder -fragment als auch an den/das Antikörper oder -fragment, der/das an den Wirkstoff bindet, zu binden.

4. Produkt nach Anspruch 3, wobei die ersten und weiteren Antikörper ganze Antikörper sind und wobei der Antikörper für den Stoff und der erste Antikörper beide von derselben Spezies abgeleitet wird und der dritte Antikörper ein Antikörper für eine weitere Spezies zu jener Spezies ist.

5. Produkt nach einem der vorangehenden Ansprüche, wobei der Wirkstoff ein Enzym ist.

EP 0 453 097 B1

**6.** Produkt nach Anspruch 5, wobei das Enzym eine Oxidase ist.

**7.** Produkt nach einem der vorangehenden Ansprüche, wobei das/die Vehiculum/Vehicula zwei Enzyme enthält/enthalten.

**8.** Produkt nach Anspruch 7, wobei die weiteren Antikörper oder Antikörperfragmente entsprechende Antikörper oder -fragmente, die zu jedem der zwei Enzyme binden und dritte Antikörper, die in der Lage sind, an entweder Antienzymantikörper oder -fragmente zu dem ersten Antikörper oder -fragment binden zu können, umfassen.

**9.** Produkt nach Anspruch 7 oder Anspruch 8, wobei die Enzyme eine Oxidase, die Peroxid erzeugt und eine Peroxidase darstellen.

**10.** Produkt nach einem der vorangehenden Ansprüche, wobei die ersten und weiteren Antikörper ganze Antikörper sind und mindestens einer der Antikörper monoklonal ist.

**11.** Produkt nach einem der vorangehenden Ansprüche, wobei das/die Vehiculum/Vehicula zur örtlichen Anwendung im Mund geeignet ist/sind.

**12.** Produkt nach Anspruch 11, wobei der erste Antikörper oder das erste Antikörperfragment ein Antikörper für S. mutans, S. sanguis, A. viscosus oder A. naeslundii ist.

**13.** Produkt nach einem der vorangehenden Ansprüche, wobei das Produkt oder mindestens ein Vehiculum durch ein Mundwasser, eine Zahnpasta oder eine Pastille bereitgestellt wird.

**14.** Verfahren zur Angabe eines kosmetischen Wirkstoffs an einen Zielort, umfassend Exposition des Zielorts mit einem Produkt gemäß einem der vorangehenden Ansprüche.

**15.** Verfahren zur Angabe eines kosmetischen Wirkstoffs an einen Zielort, umfassend Expositiopn des Ortes einem oder mehreren Vehicula, die in denselben oder getrennten Vehicula enthalten:
i) einen ersten Antikörper oder ein erstes Antikörperfragment zur Bindung durch Antikörper-Antigenbindung an einen Zielort,
ii) einen oder mehrere weitere Antikörper oder Antikörperfragmente, die in der Lage sind, gemeinsam durch Antikörper-Antigenbindung sowohl an einen Wirkstoff, als auch an den/das erste(n) Antikörper oder -fragment zu binden, wodurch das Mittel an den Zielort gebunden wird.

**16.** Verwendung eines Antikörpers oder -fragmentes, ausgewiesen in einem der Ansprüche 1 bis 13 zur Herstellung eines Produkts zur örtlichen Anwendung, um einen Wirkstoff an einen Zielort zu binden.

**Revendications**

**1.** Produit comprenant un ou plusieurs véhicules contenant, dans le même véhicule ou dans des véhicules distincts:
I) un premier anticorps ou fragment d'anticorps destiné à se fixer, par liaison antigène-anticorps, à un site cible,
II) un ou plusieurs autres anticorps ou fragments d'anticorps capables collectivement de se fixer, au moyen de liaison antigène-anticorps, à la fois à un agent actif et au premier anticorps ou fragment, pour ainsi fixer l'agent au site cible.

**2.** Produit selon la revendication 1, qui comprend en outre l'agent actif.

**3.** Produit selon la revendication 1 ou 2, dans lequel les autres anticorps ou fragments sont un anticorps ou fragment d'anticorps qui se fixe à l'agent actif et un troisième anticorps ou fragment capable de se fixer à la fois audit premier anticorps ou fragment et à l'anticorps ou fragment qui se fixe à l'agent actif.

**4.** Produit selon la revendication 3, dans lequel lesdits premier et autres anticorps sont des anticorps complets et dans lequel l'anticorps fixé à l'agent et le premier anticorps proviennent tous deux de la même espèce et le troisième anticorps est un anticorps provenant d'une autre espèce que celle-ci.

17

**5.** Produit selon l'une quelconque des revendications précédentes, dans lequel l'agent actif est une enzyme.

**6.** Produit selon la revendication 5, dans lequel l'enzyme est une oxydase.

**7.** Produit selon l'une quelconque des revendications précédentes, dans lequel le(s)dit(s) véhicule(s) contient deux enzymes.

**8.** Produit selon la revendication 7, dans lequel les autres anticorps ou fragments d'anticorps comprennent des anticorps ou fragments respectifs se fixant à chacune des deux enzymes et un troisième anticorps capable de lier l'un ou l'autre des anticorps ou fragments anti-enzymes audit premier anticorps ou fragment.

**9.** Produit selon la revendication 7 ou 8, dans lequel les enzymes sont une oxydase qui engendre un peroxyde et une peroxydase.

**10.** Produit selon l'une quelconque des revendications précédentes, dans lequel lesdits premier et autres anticorps sont des anticorps complets et au moins un des anticorps est monoclonal.

**11.** Produit selon l'une quelconque des revendications précédentes, dans lequel le(s) véhicule(s) est/sont approprié(s) à l'application locale dans la cavité buccale.

**12.** Produit selon la revendication 11, dans lequel le premier anticorps ou fragment d'anticorps est un anticorps dirigé contre *S. mutans*, *S. sanguis*, *A. viscosus* ou *A. naeslundii*.

**13.** Produit selon l'une quelconque des revendications précédentes, dans lequel le produit est, ou au moins un véhicule est fourni par, un bain de bouche, une pâte dentifrice ou une pastille.

**14.** Procédé d'apport d'un agent cosmétiquement actif à un site cible, comprenant l'exposition du site cible à un produit selon l'une quelconque des revendications précédentes.

**15.** Procédé d'apport d'un agent cosmétiquement actif à un site cible, comprenant l'exposition du site à un ou plusieurs véhicules contenant, dans le même véhicule ou dans des véhicules distincts:
I) un premier anticorps ou fragment d'anticorps destiné à se fixer, par liaison antigène-anticorps, à un site cible,
II) un ou plusieurs autres anticorps ou fragments d'anticorps capables collectivement de se fixer, au moyen de liaison antigène-anticorps, à la fois à un agent actif et au premier anticorps ou fragment, pour ainsi fixer l'agent au site cible.

**16.** Utilisation d'au moins un anticorps ou fragment tel que défini dans l'une quelconque des revendications 1 à 13, pour la préparation d'un produit destiné à l'application locale, afin de fixer un agent actif à un site cible.

Fig.1.

Fig.2.

Fig. 3.

Fig.4.

# Fig. 5.